# EUROPEAN PATENT SPECIFICATION

(11) **EP 0 614 369 B1**
(45) Date of publication and mention of the grant of the patent: **29.12.1999**
(21) Application number: 92922891.4
(22) Date of filing: 21.10.1992
(51) Int. Cl.: C07K 5/06, C07K 5/10, C07K 5/12, C07K 7/64, A61K 38/02

(54) **PHARMACEUTICAL DIPEPTIDE COMPOSITIONS AND METHODS OF USE THEREOF**
Pharmazeutische Dipeptid Zusammensetzungen und Verwendungsmethoden.
COMPOSITIONS PHARMACEUTIQUES DIPEPTIDIQUES ET LEURS PROCEDES D'UTILISATION

(30) Priority: 28.10.1991 US 783518
(43) Date of publication of application: 14.09.1994
(73) Proprietor: CYTRAN LTD., Hamilton HM11 (BM)
(72) Inventor: KHAVINSON, Vladimir Khatskelevich, St. Petersburg, 197198 (RU); MOROZOV, Vyacheslav Grigorievich, St Petersburg, 197198 (RU); SERY, Sergey Vladimirovich, St. Petersburg, 193012 (RU)
(74) Representative: Beresford, Keith Denis Lewis
(86) International application number: US9209114
(87) International publication number: WO9308815

(56) References cited:
- EP-A- 0 346 501
- WO-A-92/17191
- US-A- 4 752 602
- EXPERIENTIA vol. 42, no. 5 , May 1986 , BASEL CH pages 521 - 530 G.H. WERNER ET AL. 'Immunomodulating peptides'
- Medline Abstracts, issued 1990, RODIONOV et al., "The immunocorrective Therapy of Pyoderma Caused by Staphylococci Multiply Resistant to Antibiotics", Abstract No. 90224329, Vestn. Dermatol. Venerol., Volume 1, pages 42-45, see Abstract.
- Medline Abstract, issued September 1991, IAKOVLEV et al., "The Biochemical and Immunological Indices in the Rehabilitation Period of the Victims of the Accident on the Komsomolets Atomic Submarine", Abstract No. 92101433, Voen. Med. Zh, Volume 9, pages 28-33, see Abstract.
- Bulletin: "Thymogen", published 1989 by Cytomed (Lenningrad), pages 3-10.
- Medline Abstract, issued 1991, KHMEL'NITSKII et al., "Morphofunctional Characteristics of the Immunocompetent System in Hypotrophy and its Correction by Thymogen", Abstract No. 92171772, Arkh. Patol. Volume 53(10), pages 24-27, see Abstract.
- Medline Abstract, issued September-October 1991, GRIGORIANTS et al., "Immunocorrection in the Combined Treatment of Patients with Osteomyelitis Developing Following Combined Injuries to the Maxillofacial and Craniocerebral Areas", Abstract No. 92188368, Stomatologia(Mosk), Volume 5, pages 53-54, see Abstract.
- Biological Abstract, Volume 92, issued 1991, RODIONOV et al., "Natural Killer Activity in Patients with Chronic Dermatoses", Abstract No. 100385, Vestn. Dermatol. Venerol. Volume 5, pages 4-6, see Abstract.
- CHEMICAL ABSTRACTS, Volume 116, No. 17, issued 1990, ALIEV et al., "Simulation of Thymus Dysfunction in Guinea Pigs by using Immunomodulators", Abstract No. 171986U, Izv. Akad. Nauk. Az. Ssr, Ser. Biol. Nauk., Volume 1, pages 73-80, see Abstract.
- CHEMICAL ABSTRACTS, Volume 116, No. 17, issued 1991, DEMIDOV et al., "Effects of Thymus Preparations and Antituberculous Drugs on Immunological Reactivity and the Course of Tuberculous Process in Experimental Animals", Abstract No. 165824Y, Probl. Tuberk. Volume 12, pages 52-54, see Abstract.

## Description

The present invention is directed to dipeptide polymer pharmaceutical compositions and uses thereof, in particular, uses thereof for treatment of immunodepressed states and of opportunistic infections in immunodepressed states.

### BACKGROUND OF THE INVENTION

Several polypeptides found in the thymus gland have been implicated as playing roles in the development and maintenance of immunological competence in animals, including human beings. Some of these polypeptides have been shown to stimulate the maturation, differentiation and function of T-cells. For example, a heat-stable fraction isolated from calf thymus extracts, designated as Thymosin fraction 5, has been shown to reconstitute immune functions in thymic-deprived or immunodepressed individuals. Several peptides have been isolated from Thymosin fraction 5, such as Thymosin alpha₁ (28 amino acids, U.S. Patent No. 4,079,127), Thymosin beta₄ (44 amino acids, Low et al., PNAS, 78,1162-1166 (1981)), Thymosin betas (39 amino acids, U.S. Patent No. 4,389,343) and Thymosin beta₉ (41 amino acids, U.S. Patent No. 4,389,343). However, practical administration of such polypeptides is expensive due to the relatively low yield and complexity of isolation and/or manufacture of such long chain polypeptides. Most importantly, in some cases, these polypeptides produce side reactions in patients.

A dipeptide Glu-Trp, hereinafter referred to as Thymogen, exhibits a broad range of efficacy for prevention and treatment of opportunistic infections in immunodepressed states, and for therapeutically effective treatment of immunodeficient states. This is believed to be highly unexpected for such a relatively small compound to exhibit such a broad range of activity. Furthermore, we have not found any significant side effects from the use of the dipeptide according to the present invention. Due to its simple nature, the dipeptide is rather inexpensive to manufacture. EP-A-0346501 also discloses the above dipeptide and its use in the treatment of immune-deficient states.

AS used herein, the terms "immunomodulator" and "immunomodulating" encompass the activity of enhancing or restoring the subject's immune system, as evidenced by measurable blood parameters and/or the patient's improved ability to combat infection or disease, and the ability to heal tissue. Hence, immunomodulation encompasses improvement of the immune system due to an immunodeficient state (for example, caused by removal of the thymus), and/or an immunodepressed state (for example, caused by exposure to radiation). Furthermore, the present invention provides for modulation of the immune system by lowering blood parameters and other indicia of the immune state if these indicia are abnormally elevated. The present invention encompasses the therapeutic method of treating the immunodeficient, immunodepressed or elevated immune state per se, thus providing prophylaxis against infection and disease, as well as a treatment of infection, disease or wound indirectly by enhancing the immune system.

In one aspect, the invention provides a substantially pure compound as defined in claim 27 of the accompanying claims.

In another aspect the invention provides a pharmaceutical composition as defined in claim 22 or 23 of the accompanying claims.

In another aspect the invention provides uses of peptide polymers for the manufacture of pharmaceutical preparations as set out in claims 1, 5, 7, 8, 12, 14, 16, 18, 19 and 21 of the accompanying claims.

### DESCRIPTION OF THE PREFERRED EMBODIMENTS

The compositions containing the dipeptide polymers according to the present invention may be formulated into any convenient formulation which allows for the active ingredient to be absorbed into the blood stream. Intramuscular and intranasal forms of application are preferred. The preferred dosage rate of the active ingredient for intramuscular administration is about 50 to 100µg per dose for adults (for a 300 to 1000µg total treatment therapy); for infants up to 1 year old about 10µg per dose, for infants 1 to 3 years old about 10 to 20µg per dose; for infants 4 to 6 years old about 20 to 30µg per dose, for children 7 to 14 years old about 50µg per dose. All of the foregoing dosages are useful for a treatment of 3 to 10 days, depending upon the immunodeficiency level. The treatment may be repeated as needed, usually within 1 to 6 months.

For prophylactic uses against opportunistic infections in immunodeficient or immunodepressed patients, the intramuscular and/or intranasal single daily dose for adults may be from about 50 to 100µg, and for children about 10 to 50 µg per dose for treatment over 3 to 5 days.

For treatment of burns, frost bite, or other wounds, including chronic apical periodontitis, the dipeptide may be applied in about 100µg doses as a paste or other suitable medium.

For ophthalmology, such as for treatment of infectious eye diseases, the dipeptide may be applied in single daily dosages of about 10µg (over 4 to 10 days) or as installations into the conjunctival cavity at about 5µg twice daily over about 4 to 5 days.

The dipeptide polymer may be utilized intramuscularly as an injection solution with the active ingredient in a therapeutically effective immunopotentiating amount of about .001 to .01% by weight. If presented in the form of a tablet, capsule or suppository it is preferred that the active ingredient be present in an amount of about 0.1mg per tablet, suppository or capsule. If presented in such form, the capsule, suppository or tablet may also contain other conventional excipients and vehicles such as fillers, starch, glucose, etc.

The dipeptisde L-Glu-L-Trp may be obtained by conventional peptide synthesis, including the Merrifield solid state peptide synthesis technique. Typically an amino and side chain protected derivative of an activated ester of glutamic acid is reacted with protected L-tryptophan. After elimination of the protecting groups and conventional purification, such as by thin layer or GL chromatography, the peptide may be purified such as by, lyophilization, gel purification, and the like.

The purified dipeptide L-Glu-L-Trp, comprises a white powder (if lyophilized; otherwise, it is crystalline), soluble in water, DMF; insoluble in chloroform and ether. [alpha²²_{D} = +12.6; C = 0.5 H₂O. R_{f} = 0.65 (butanol: acetic acid: water = 3:1:1). UV (275 ± 5nm, max). NMR (500MHz): 0.001mol/l of the peptide solution, Trp (3.17; 3.37; 4.57; 7.16; 7.24; 7.71; 7.49); Glu (1.90; 1.96; 2.21; 3.72).

Other forms of the dipeptide including the cyclized monomer. 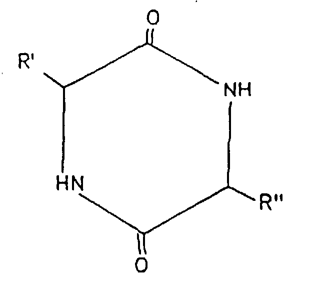 Wherein R' and R" are, respectively , the alpha-side chains of Glu and Trp;

The invention concerns linear polymers 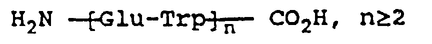 and cyclic polymers

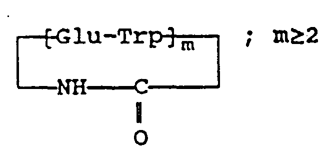

The linear polymers may be made by conventional peptide systhesis, including Merrifield solid-state peptide methodology. The cyclic polymers may be prepared by cyclizing the linear peptide in with peptide linkage agents in dilute solutions.

The active dipeptide polymer ingredient of the pharmaceutical preparations according to the present invention may be used as a free peptide polymer or in the form of a water soluble pharmaceutically acceptable salt, such as a sodium, potassium, ammonium or zinc salt. It will be understood that the dipeptide polymer may be administered with other active ingredients which independently impart an activity to the composition, such as, antibiotics, interferon, anesthetics, and the like.

The most preferred formulation according to the present invention is a solution for intramuscular injection containing about .001 to .01% by weight (.0001-.001mg/kg body weight, or 10-100µg active ingredient per lml solvent). The pharmaceutically acceptable vehicle for this injection form may be any pharmaceutically acceptable solvent such as 0.9% aqueous sodium-chloride, distilled water, Novocaine solution, Ringer's solution, glucose solution, and the like. The dipeptide polymer containing compositions according to the present invention may be administered in a compatible pharmaceutical suitable for parenteral administration (e.g., intravenous, subcutaneous, intramuscular). The preparations may be subjected to conventional pharmaceutical operations, such as sterilization, and may contain adjuvants, such as preservatives, stabilizers, wetting agents and the like.

The pharmaceutical preparations according to the present invention demonstrate a high effectiveness in the treatment of immunodepressed and immunodeficient states for the preventing and treatment of opportunistic infections in those states.

Also included within the scope of the present invention are the pharmaceutically acceptable salts of the dipeptide polymer, such as sodium or potassium or strong organic bases, such as guanidine.

The dipeptide polymer containing compositions according to the present invention have activity in the restoration and stimulation of the immune functions. Thus they are useful in the treatment of opportunistic infections of an immunodepressed subject in an immunopotentiating effective amount as described above.

The dipeptide polymer compositions according to the present invention may also be used in veterinary practice as an immunomodulatory agent for prophylaxsis and treatment of hypotrophy in farming animals, fur bearing animals and poultry.

Among the opportunistic infections which may be treated utilizing the compositions according to the present invention are: respiratory diseases, influenza, AIDS, burns, wounds, other open sores, rashes (due to allergic reactions), sun exposure, local trauma (with an ointment), eczemas, psoriasis, and the like. Furthermore, the compositions according to the present invention may be utilized to assist healing in immunodepressed or immunodeficient states, such as for the healing of bone fractures, lesions, gingival diseases, gynecological infections, infralymphatic infections, and the like. The compositions may also be used to enhance the immunodeficient state to increase susceptibility to microbial antibiotics and to enhance the patient's responsive reaction to other types of therapies.

The compositions according to the present invention also may be utilized to enhance metabolic processes; to enhance production of blood insulin; for treatment of irradiated cancer patients, as well as for veterinary uses.

Another important use is the treatment of chronic fatigue syndrome (CFS), which is believed to be a manifestation of an immunodepressed state.

The following examples are provided for reference purposes to demonstrate the clinical effectiveness of the compound Thymogen, of which the materials claimed herein are polymers.

### EXAMPLE 1

About 262 adult patients were treated over a period of 2 months on a daily basis with intramuscular injections of solutions containing 100 µg of Thymogen. These patients were treated for 2 months immediately succeeding exposure to radiation caused from the Chernobyl nuclear accident. As a control, about 18 people exposed to radiation were tested for various blood parameters to establish a baseline.

The results are shown below.

| THE EFFICIENCY OF RADIATION IMMUNODEFICIENCY CORRECTION TWO MONTHS AFTER IRRADIATION (X±m) | | | |
|---|---|---|---|
| Indices | Examinated groups | | |
| | Healthy (control) | Irradiated | |
| | | Prior to therapy | After THYMOGEN therapy |
| Leukocytes, abs | 5.6±0.8 | 3.5±0.4* | 5.0±1.2** |
| Lymphocytes, abs | 1.98±0.16 | 0.80±0.24* | 1.9±0.4** |
| CD2-DR+, % | 35.8±0.9 | 21 ± 4* | 30.0±1.2** |
| CD2-DR+, abs | 0.59±0.04 | 0.16±0.04* | 0.55±0.06* |
| CD2, % | 49.3±1.5 | 32 ± 7 | 48.7±1.8** |
| CD2, abs | 0.98±0.09 | 0.55±0.08* | 1.13±0.08** |
| E-RFC, % | 30.2±1.6 | 22.9±1.9* | 27.4±2.4** |
| LMI with ConA, % | 65.0±2.1 | 120 ± 17* | 90 ± 10** |
| CD19, % | 22.0±1.7 | 32 ± 3* | 27 ± 4 |
| CD19, abs | 0.46±0.03 | 0.26±0.06* | 0.51±0.10** |
| IgM, g/l | 1.1 ± 0.4 | 0.87±0.07 | 1.00±0.10 |
| IgG, g/l | 11.1±0.9 | 10.2±2.0 | 10.0±1.0 |
| IgA, g/l | 1.70±0.10 | 1.5±0.4 | 1.49±0.19 |

| | | | |
|---|---|---|---|
| * - statistically significant (P<0.05) vs. the indices in healthy people; | | | |
| ** - statistically significant (P<0.05) vs. the data obtained prior to immunocorrection with THYMOGEN; abs - cell concentration presented as 109_{/l;} LMI - leucocyte migration inhibition; RFC - rosette-forming cells. | | | |

### EXAMPLE 2

The group of patients described in Example 1 were further treated for a period of 36 months and tested again subsequent to the first stage of therapy (after 4 months) and after the second stage of therapy (6 months). The blood parameters are shown below. As can be seen most of the blood parameters were elevated after both the first and second stages of therapy.

| THE PROLONGED THYMOGEN THERAPY TRIALS RESULTS IN IRRADIATED PATIENTS (X±m) | | | |
|---|---|---|---|
| Indices | Interims of examination | | |
| | prior to therapy | after the 1st stage of THYMOGEN use | after the 2nd state of THYMOGEN use |
| Leucocytes, abs | 3.5±0.5 | 4.7±0.2* | 5.5±0.3* |
| Lymphocytes, abs | 1.0±0.5 | 1.5±0.4 | 1.9±0.5* |
| CD2-DR+, % | 12.8±2.6 | 22.3±0.5 | 29 ± 3* |
| CD2-DR+, abs | 0.13±0.04 | 0.34±0.05* | 0.56±0.08* |
| CD3, % | 24 ± 3 | 35 ± 4* | 46 ± 3* |
| CD3, abs | 0.26±0.05 | 0.49±0.06* | 0.89±0.11* |
| CD4, % | 7.1±1.1 | 19.5±1.7* | 24.1±1.5* |
| CD4, abs | 0.07±0.01 | 0.28±0.03* | 0.45±0.04* |
| CD8, % | 17 ± 3 | 15.4±2.3 | 22.3±2.2* |
| CD8, abs | 0.16±0.04 | 0.23±0.03 | 0.40±0.05* |
| CD19, % | 12.2±1.9 | 15.0±2.8 | 21.1±2.1* |
| CD19, abs | 0.14±0.04 | 0.21±0.06 | 0.39±0.06* |

| | | | |
|---|---|---|---|
| * - statistically significant (P<0.05) in comparison with the indices prior to therapy; abs - cell concentration presented as 10⁹/l. | | | |

### EXAMPLE 3

The patients described in Example 1 were tested for blood parameters the first few days after exposure to the radiation of the Chernobyl accident. It could be seen from the table below that response to the treatment was observed even after a few weeks? of treatment.

| THYMOGEN INFLUENCE ON IMMUNE STATUS IN EARLY TERMS AFTER IRRADIATION AFFECTION (X±m) | | | |
|---|---|---|---|
| Indices | Examinated groups | | |
| | Healthy | Irradiated | |
| | (control) | Prior to therapy | After THYMOGEN therapy |
| Leukocytes, abs | 5.7±0.3 | 3.8±0.3* | 6.4±0.8** |
| Lymphocytes, abs | 1.91±0.12 | 1.15±0.14* | 2.27±0.16** |
| CD2-DR+, % | 30.8±1.1 | 17.6±2.O* | 31 ± 3** |
| CD2-DR+, abs | 0.59±0.04 | 0.20±0.03* | 0.69±0.08** |
| CD2, % | 50.6±1.6 | 47 ± 4 | 50.9 ± 2.4 |
| CD2, abs | 0.98±0.09 | 0.55±0.08* | 1.13±0.07** |
| E-RFC, % | 29.7±2.5 | 29.8±2.6 | 23.4±2.6 |
| LMI with ConA, % | 66 ± 4 | 98 ± 9* | 60 ± 7** |
| CD19, % | 22.8±2.2 | 27.0±2.8 | 30.5±1.9* |
| CD19, abs | 0.47±0.03 | 0.30±0.05* | 0.68±0.04** |
| IgM, g/l | 1.1±0.4 | 0.51±0.08* | 0.58±0.10* |
| IgG, g/l | 10.1±0.9 | 8.6±1.3 | 9.2 ±0.7 |
| IgA, g/l | 1.71±0.16 | 2.07±0.20 | 1.11±0.09*,** |
| C3, g/l | 0.57±0.03 | 0.74±0.07 | 0.68±0.04 |

| | | | |
|---|---|---|---|
| * - statistically significant (P<0.05) in comparison with the indices in healthy people | | | |
| ** - statistically significant (P<0.05) in comparison with the data obtained prior to THYMOGEN use; LMI - leukocyte migration inhibition; abs - cells concentration presented as 10⁹/l. | | | |

### EXAMPLE 4

A number of (36) breast cancer patients were treated with the Thymogen by injection of daily dosages of 100 µg (a.i.). The patients had been previously treated with radiation therapy (single doses 2 grad; total dose 45-50 grad). It can be seen from the table below the treatments restore their blood parameter levels.

| IMMUNITY AND NON-SPECIFIC RESISTANCE INDICES IN BREAST CANCER PATIENTS TREATED WITH THYMOGEN AFTER RADIOTHERAPY (Xtm) | | | |
|---|---|---|---|
| Indices | Prior to radiotherapy | After radiotherapy | After THYMOGEN use |
| Lymphocytes (x10⁹/l) | 1.61±0.18 | 0.79±0.09* | 1.72±0.21** |
| T-lymphocytes (x10⁹/l) | 0.83±0.07+ | 0.32±0.03* | 0.92±0.12** |
| "Active" T-lymphocytes (x10⁹/l) | 0.49±0.06 | 0.19±0.03* | 0.52±0.07** |
| T-helpers (OKT4⁺) (x10⁹/l) | 0.30±0.03 | 0.12±0.01* | 0.39±0.04** |
| T-suppressors (OKT8⁺) (x10⁹/l) | 0.28±0.04 | 0.16±0.02* | 0.21±0.03 |
| OKT4⁺/OKT8⁺ | 1.07±0.09 | 0.75±0.06* | 1.86±0.17** |
| DSH^{a} to tuberculin (mm) | 7.3t0.4 | 2.6±0.2* | 8.7±0.6** |
| LMI^{b} with ConA (%) | 68 ± 4 | 96 ± 7* | 71 ± 5** |
| SI^{c} to THYMOGEN | 1.23±0.15 | 1.19±0.13 | 1.27±0.14** |
| B-lymphocyte (Ig⁺) (x10⁹/l) | 0.15±0.02 | 0.11±0.01 | 0.17±0.02 |
| Phagocytic index | 4.3±0.3 | 2.06±0.18* | 3.7±0.2** |
| Cation proteins | 1.58±0.09 | 1.36±0.08* | 1.49±0.12 |
| C₃-complement (g/l) | 0.75±0.05 | 0.66±0.04 | 0.68±0.04 |

| | | | |
|---|---|---|---|
| * - statistically significant (P<0.05) vs. the analogous index before radiotherapy; | | | |
| ** - statistically significant (P<0.05) vs. the analogous index after radiotherapy; a - Delayed-Skin Hypersensitivity; b - Leukocyte Migration Inhibition; c - Sensitivity Index. | | | |

### EXAMPLE 5

On peripheral blood of human volunteers in vitro. Cell cultures were incubated and treated. As can be seen from the table below, after 24 hrs. incubation at concentrations of 1 µg/ml and 100 µg/ml, there was statistically no mutagenic effect in these cultures.

| THE CALCULATION OF CHROMOSOME STRUCTURAL DAMAGES IN HUMAN PERIPHERAL BLOOD LYMPHOCYTES | | | | | |
|---|---|---|---|---|---|
| Dose of the medicine | Number of analyse d metaphases | Metaphases with chromosome structural aberrations | | Index of reliability (P) | Level of mutagenic effect (numbers) |
| | | # | % | | |
| Control | 1000 | 15 | 1.5 | - | - |
| THYMOGEN - 1 µg/ml | 1000 | 15 | 1.5 | >0.05 | 0 |
| THYMOGEN - 100 µg/ml | 1000 | 16 | 1.6 | >0.05 | 0 |

### EXAMPLE 6

About 263 patients were treated with doses of 100 µg of Thymogen introduced intramuscularly on a daily basis over a period of 3 years after exposure to the radiation at the Chernobyl accident. Blood parameters after 3 years of such treatment were restored to the statistical norm prior to their exposure to the radiation.

| Indices | Statistical norma | Results of victims examination | | |
|---|---|---|---|---|
| | | Prior to therapy | After therapy | |
| | | | THYMOGEN | Conventional |
| Leucocytes, abs | 5.2±0.2 | 5.8±0.3 | 5.6±0.4 | 5.5±1.0 |
| Lymphocytes, abs | 1.96±0.06 | 2.0±0.3 | 2.1±0.3 | 1.8±0.23 |
| CD2-DR+, % | 30.8±1.1 | 15 ± 3* | 32 ± 3** | 18.4±2.5 |
| CD2-DR+, abs | 0.59±0.04 | 0.30±0.06* | 0.66±0.10** | 0.34±0.11 |
| CD3, % | 55.6±1.9 | 67.7±2.7* | 59.2±2.1** | 61 ± 3* |
| CD3, abs | 1.09±0.08 | 1.33±0.05* | 1.21±0.15 | 1.12±0.18 |
| CD4, % | 35.3±2.7 | 36.7±2.6 | 36.2±1.7 | 38 ± 3 |
| CD4, abs | 0.69±0.05 | 0.72±0.05 | 0.74±0.08 | 0.70±0.05 |
| CD8, % | 21.3±0.9 | 29.7±0.9* | 23.2±2.1** | 25.0±2.7** |
| CD8, abs | 0.41±0.03 | 0.56±0.02* | 0.48±0.07** | 0.46±0.06** |
| T4/T8 | 1.64±0.12 | 1.24±0.10* | 1.58±0.04** | 1.52±0.13 |
| LMI, % | 59.7±1.7 | 140 ± 30* | 75 ± 6** | 107 ± 10** |
| B-Ig+, % | 13.8±1.2 | 10.7±0.3 | 11.0±0.3 | 11.2±0.7 |
| B-Ig+, abs | 0.29±0.02 | 0.21±0.01 | 0.23±0.04 | 0.20±0.05 |
| B-IgM+, % | 6.4±0.7 | 3.0±0.3* | 4.1±0.6* | 4.4±0.3 |
| B-IgM+, abs | 0.12±0.01 | 0.062±0.002 | 0.12±0.003* | 0.08±0.004 |
| B-IgG+, % | 4.1±0.5 | 4.7±0.9 | 4.8±0.5 | 4.6±0.5 |
| B-IgG+, abs | 0.078±0.008 | 0.059±0.003 | 0.09±0.007 | 0.08±0.006 |
| B-IgA+, % | 2.2±0.2 | 2.3±0.3 | 1.9±0.3 | 1.98±0.09 |
| B-IgA+, abs | 0.041±0.004 | 0.048±0.006 | 0.04±0.002 | 0.04±0.002 |
| IgM, g/l | 1.15±0.06 | 0.53±0.09* | 1.06±0.06** | 1.03±0.13** |
| IgG, g/l | 11.5±0.5 | 13.2±1.1 | 10.9±1.3 | 11.3±1.2 |
| IgA, g/l | 1.90±0.08 | 0.82±0.25 | 1.2±0.4* | 1.1±0.3 |

| | | | | |
|---|---|---|---|---|
| * - statistically significant (P<0.05) vs. the indices in healthy persons; | | | | |
| * - statistically significant (P<0.05) vs. the data obtained prior to therapy; abs - cell concentration presented as 10⁹/l; LMI - leucocyte migration inhibition. | | | | |

### EXAMPLE 7

The patients described above in Example 6 were tested for blood parameters after 6 months of treatment immediately following exposure to the radiation caused by the Chernobyl accident. The results below show that after 6 months those treated with Thymogen showed improvement over those patients who were not treated.

| THYMOGEN USE EFFICIENCY IN ACUTE RADIATION SICKNESS (6 MONTHS AFTER ACCIDENCE) (X±m) | | | | |
|---|---|---|---|---|
| Indices | Healthy people (statistic norma) | Patients (suffered in accidence) | | |
| | | Prior to therapy | After therapy | |
| | | | Without THYMOGEN | With THYMOGEN |
| Lymphocytes, % | 33.9±1.2 | 32.9±2.4 | 29.2±2.0 | 30.0±1.8 |
| Lymphocytes, abs | 1.96±0.06 | 1.49±0.14* | 1.39±0.13 | 1.52±0.12* |
| CD2, % | 53.6±1.9 | 38.7±2.7* | 32 ± 3* | 49 ± 3** |
| CD2, abs | 1.05±0.05 | 0.56±0.04* | 0.44±0.04* | 0.75±0.05* ,** |
| CD2-DR+, % | 30.8±1.1 | 18.9±1.6* | 19.7±1.2* | 20.8±1.6^{*},^{**+} |
| CD2-DR+, abs | 0.59±0.04 | 0.30±0.25* | 0.28±0.02* | 0.31±0.02* ,**⁺ |
| CD3, % | 55.6±1.9 | 39.0±2.4* | 37 ± 5* | 53.4±1.8** |
| CD3, abs | 1.09±0.08 | 0.58±0.04* | 0.51±0.03* | 0.82±0.04** |
| CD4, % | 35.3±2.7 | 20.3±1.3* | 18.9±1.3* | 32.6±1.4** |
| CD4, abs | 0.69±0.05 | 0.30±0.03* | 0.26±0.03* | 0.50±0.04** |
| CD8, % | 21.3±0.9 | 19.5±1.5 | 17.5±1.6 | 21.2±1.8 |
| CD8, abs | 0.41±0.03 | 0.29±0.03 | 0.24±0.03 | 0.32±0.113 |
| T4/T8 | 1.64±0.12 | 1.04±0.04* | 1.08±0.10* | 1.54±0.11** |
| LMI | 59.7±1.7 | 106 ± 6* | 107 ± 6* | 72.7±4.5* ,** |
| CD19, % | 25.00±0.12 | 18.2±2.1* | 23 ± 3 | 26.7±2.1 |
| CD19, abs | 0.49±0.04 | 0.27±0.03* | 0.31±0.05* | 0.41±0.03** |
| B-Ig+, % | 13.8±1.2 | 15.8±1.3 | 16.2±1.7 | 19.0±1.3* ,** |
| B-Ig+, abs | 0.29±0.02 | 0.23±0.03 | 0.23±0.04 | 0.29±0.03 |
| B-IgM+, % | 6.4±0.7 | 6.3±0.8 | 5.4±0.5 | 8.8±0.7 |
| B-IgM+, abs | 0.12±0.01 | 0.09±0.01* | 0.08±0.01* | 0.13±0.02 |
| B-IgG+, % | 4.1±0.5 | 7.8±0.9* | 7.1±0.8* | 6.4±0.5* |
| B-IgG+, abs | 0.082±0.008 | 0.098±0.007 | 0.104±0.008 | 0.100±0.007 |
| B-IgA+, % | 2.20±0.20 | 1.80±0.15* | 1.70±0.20* | 1.8±0.3 |
| B-IgA+, abs | 0.038±0.004 | 0.033±0.004 | 0.024±0.003 | 0.030±0.002 |
| IgM, g/l | 1.15±0.06 | 1.14±0.08 | 1.20±0.07 | 1.07±0.09 |
| IgG, g/l | 11.5±0.5 | 11.9±1.0 | 11.7±0.9 | 10.9±1.1 |
| IgA, g/l | 1.9±1.0 | 1.6±0.8 | 1.6±0.8 | 1.8±0.9 |

| | | | | |
|---|---|---|---|---|
| * - statistically significant (P<0.05) vs. the indices in healthy people; | | | | |
| ** - statistically significant (P<0.05) vs. the data obtained before immunocorrection; abs - cell concentration presented as 10⁽/l; LMI - leucocyte migration inhibition. | | | | |

### EXAMPLE 8

A group of 452 persons were treated with daily dosages of 100 µg of Thymogen administered intramuscularly over a period of 5-10 days and compared with a random group (250 persons) (not similarly treated) as a control. The cases of respiratory diseases and influenza were recorded for both groups. As can be seen from the table below, the untreated group had a greater occurrence of the diseases and sicknesses, hospitalization or disablement than the group treated with Thymogen.

| THYMOGEN CLINICO-EPIDEMIOLOGICAL PREVENTIVE EFFICIENCY IN ARD AND FLU | | | |
|---|---|---|---|
| Indices | Group of observation | | Index of efficiency |
| | THYMOGEN | Control | |
| Sickness rate per 100 persons/month | 9.8 | 30.4 | 3.1 |
| Pneumonia rate/100 persons/month | 0.20 | 0,50 | 2.5 |
| Need in hospitalization, % | 30.6 | 44.9 | 1.7 |
| Average term of hospitalization, days | 6.2 | 8.8 | 1.4 |
| Rate of lingering and complicated cases, % | 3.9 | 13.8 | 3.5 |
| The same index in in-patients, % | 9.8 | 26.2 | 2.7 |
| Number of temporary disablement cases/100 persons/month | 4.1 | 7.0 | 1.7 |
| Number of temporary disablement days/100 persons/month | 26.5 | 57.6 | 2.2 |

| THE DYNAMICS OF ARD AND INFLUENZA RATE IN GROUPS OF OBSERVATION BY MONTHS FROM THE BEGINNING OF INVESTIGATION | | | | | |
|---|---|---|---|---|---|
| Indices | Groups | 1st month | 2nd month | 3rd month | 4th month |
| Sickness rate/100 persons/month | THYMOGEN Control | 9.6 28.6 | 11.3 33.4 | 9.4 28.7 | 11.0 30.6 |
| Need in hospitalization, % | THYMOGEN Control | 27.0 41.2 | 27.1 50.8 | 28.3 48.8 | 28.3 39.0 |
| Average term of hospitalization, days | THYMOGEN Control | 6.2 9.3 | 6.2 8.4 | 6.2 10.2 | 7.0 11.0 |
| Number of temporary disablement cases/100 persons/month | THYMOGEN Control | 3.8 6.9 | 7.3 10.2 | 3.4 7.5 | 3.6 5.6 |
| Number of temporary disablement days/100 persons/month | THYMOGEN Control | 24.0 47.9 | 48.6 82.0 | 14.3 51.4 | 26.3 42.8 |

### EXAMPLE 9

In separate studies, a total of 21 AIDS infected individuals have been studied, including full-blown syndrome, prodromal, and pre-AIDS afflicted individuals who were treated with Thympentin. Thympentin and TPI are thymic gland peptide extracts previously well characterized. Comparative studies between TPI and Thymogen reveal that Thymogen is a far more effective cell mediator, restoring normal immunologic indices, including T-cell functional activity and T4/T8 ratios. Method of Administration: Sterile saline containing the sodium salt of the medication is administered either IM, infralymphatically, or intranasally each day for 5 - 10 days consecutively every 30 days.
Immunosupressed individuals who have sustained radiation injuries were treated with Thymogen with excellent restoration of immunological indices and models for acquired immune deficiency syndrome. Thymogen may thus benefit AIDS infected individuals by reducing the need to use other medications with toxic side effects, and sustain and or support the individuals by reducing the needs to use other medications with toxic side effects, and sustain and or support the individuals immune indices resulting in a reduction of opportunistic infections.

### EXAMPLE 10

A total of 159 patients were treated with pyoderma, including furunculitis, cellulitis, and folliculitis, with a control group consisting of 25 patients who were not treated with thymogen. Medications were administered either IM or intranasally for 5 consecutive days. Immunological indices were normalized with disappearance of skin manifestations and relapses were prevented after treatment with Thymogen. Clinical improvement correlated with immunological indices correction. Administration IM, intranasally, or topically as a sterile saline solution of medication for a period of 5 to 10 days at a concentration of 1 µg/kg body weight.

### EXAMPLE 11

A number of patients within the group of 159 patients afflicted with furunculitis, pyoderma, cellulitis, and folliculitis were afflicted with acne vulgaris and acne. The immunological indices corrected and normalized rapidly within the group therapy. The clinical outcome correlated with the correction of immunological indices, and relapses were controlled.

### EXAMPLE 12

A total of 30 patients were treated with psoriasis and 30 patients were used as controls and were untreated with thymogen. All patients had at a least 5 year history of no unsuccessful treatment. The administration of 100 µg IM or intranasally for a period of 10 days resulted in the improvement in 7% of the patients, significant improvement in 60% of patients, and total recovery in 33% of the patients.

### EXAMPLE 13

A total of 46 female patients with the various disorders (pelvic inflammatory diseases, cervicitis, vaginitis and various tubo-ovarian and adnexal abscesses) were treated and 50 patients were used as controls. Thymogen was applied IM, intranasally at 100 µg 5 consecutive days or 50 µg intralymphatically for 5 consecutive days in conjunction with conventional therapy. The clinical effect of Thymogen expressed the arresting of pain syndrome, the control of body temperature, e.g. reduction of fever, the decrease of duration of conventional treatment. The normalization of immune status correlated with clinical improvements.

### EXAMPLE 14

Patients treated with Thymogen either topically, IM, or intranasally experienced marked reduction of recurrence of herpetic lesions, with substantial reduction in the period between outbreaks. In one study, individuals who experienced 7-10 outbreaks per year experienced less than one outbreak per year after treatment with Thymogen in combination with interferon.

### EXAMPLE 15

A total of 37 patients with Herpes Zoster were treated with Thymogen in combination with conventional interferon treatment and 25 control patients with interferon alone. Administration single daily IM or intranasal 100 µg during a period of 10 days resulted in accelerated regression of foci of herpetic infection. There was noted prevention of relapses, and healing occurred on the average 40% earlier than control groups. Immunological indices correlated with clinical outcome.

### EXAMPLE 16

Patients were treated for gingival disease by subcutaneous administration of thymogen in the area of the gingiva. The treatment resulted in the arresting of gingival disease. Approximately 80 patients were studied with disease and treated and an equal number were treated conventionally without Thymogen for control purposes. Administration of 100 ug Im, Subcutaneously, or by electrophoresis (whereby a small voltage charge to the gums results in a rapid transfer of medication through the gum epithelium) resulted in the arresting of bleeding, more rapid restoration of inflammatory processes, and the decrease of purulent discharge. The treatment resulted in fewer recurrences and prolongation of normal gums. It was also noted that normalization of immunologic indices was achieved with normal coagulation.

### EXAMPLE 17

The treatment with toothpaste containing Thymogen will result in a reduction of dental caries.

### EXAMPLE 18

A total of 46 patients with periapical granulomas and 28 patients with the same disease not treated with Thymogen were used for controls. Instillation of 100 µg of Thymogen into the foramen at the base of the tooth, or in the composition of the filling paste during 3 days resulted in the accelerated arrestation of the inflammatory process, reduction in pain, and increased stability of the underlying dental structures as evidenced by x-ray studies.

### EXAMPLE 19

The use of dental toothpaste containing Thymogen will result in the reduction of gingival disease and redu ion in dental caries.

### EXAMPLE 20

The use of Thymogen 100 µg IM, intranasally, or intralymphatically controls the advance of lymphangitis.

### EXAMPLE 21

A total of 186 patients with acute respiratory disease, including upper airway diseases, such as colds, were treated with Thymogen and 87 patients who were not treated with Thymogen were used as controls. Administration IM or intranasally 100 µg 3 - 7 days resulted in a milder course of the viral infection. There was noted a decrease in the specific signs of upper respiratory infections such rhinorrhea, sore throat, fever, muscle aches, headaches, and ear pain. Secondary infectious complications were diminished, and the duration of the treatment was also diminished.

### EXAMPLE 22

A total of 51 patients were treated with Thymogen with 24 patient controls, administration IM, intranasally, and installation into sinuses with 1 µg/kg dose during a period of 3 - 10 days resulted in normalization of nasal breathing, the disappearance of nasal mucous swelling, the arresting of exudates from affect sinuses, and improved general condition and immune status. The decrease of treatment duration up to 1.7 times compared to controls.

### EXAMPLE 23

Thymogen IM or intranasal accompanying conventional therapy (antibiotics) results in accelerated healing of chronic and acute ear infections.

### EXAMPLE 24

A total of 41 patients with various eye problems as described and 36 patients in control studies were treated by conventional methods with the first group receiving Thymogen in addition to the conventional treatment. Administration of Thymogen intra ocularly at 18 µg for 5 consecutive days, or as installation into conjunctival cavity as drops bid for 5 days resulted in more rapid arresting of the inflammatory process and the increase in visual acuity, and the decrease of duration of treatment.

### EXAMPLE 25

A total of 156 patients treated with Thymogen and 82 patients in the control study, were administered, medication IM or intranasally 100 µg 5 - 10 days resulting in accelerated reduction in symptom complexes including joint pain, muscle aches, fevers, chills, and upper respiratory symptoms.

### EXAMPLE 26

A total of 263 patients and 18 control patients sustained exposure to radiation injury. Thymogen was administered IM and/or intranasally 100 µg for 10 days. A repeated course may be prescribed on the basis of immunological indices, and averages every 4 to 6 months. The results of the treatment are restoration of normal or near normal immune indices with functional activity in the majority of all cases studied. There was an arresting of esthenic syndrome, and an arresting of the somatic pathological exacerbations and reduction of opportunistic infections.

### EXAMPLE 27

Thymogen administration IM or intranasally results in the improved immune parameters, functionals activity of lymphocytes and neutrophils, and reduction of post-operative complications and infections associated with bone-marrow compromise, such as, that caused from transplant or radiation exposure.

### EXAMPLE 28

A total of 29 patients afflicted with various allergies as described and 17 patients in the control group were treated with Thymogen in dose 1 µg/kg IM or intranasally for 5 - 7 days resulted in disappearance of allergic reactions.

### EXAMPLE 29

A total of 76 patients with 72 patients in control exposed to massive hemotransfusions during post-operative period were treated with Thymogen. Thymogen was administered starting from 4-6 day of post-operative period single daily IM or intranasally in does 100 µg for 5 days. None of studies patients showed clinical manifestation of alloblood rejection while in 17% of control patients the adverse hemotranfusional reactions were observed.

### EXAMPLE 30

Thymogen was applied in 76 patients treated with antibiotics for various indications who had unfavorable allergological history. Control group comprised 43 patients. Thymogen was administered IM or intranasally single daily at 100 µg for 5-10 days. In the majority of case the use of Thymogen prevented the arising of allergic reactions or promoted the less severe course of them while in the control group in 70% of patients the pronounced signs of drug intolerance was marked.

### EXAMPLE 31

Thymogen was administered to 17 patients subjected to skin grafting. The control group comprised 27 patients. Thymogen was administered IM or intranasally single daily at 50-100 µg for 5 days. In all the patients the use of Thymogen prevented the arising of infections complications and graft rejection. In control group the manifestations of rejection were determined in 8 patients.

### EXAMPLE 32

Thymogen was administered to 52 patients suffered from chronic skin diseases caused by antibioticresistant staphylococci. 42 patients with the same pathology but not treated with the immunomodulator were the control group. Thymogen was administered IM to 27 patients single daily at 100 µg for 5 days and intranasally to 25 patients in the same daily and total dose. The differences between these two methods of application were not noticed. In all the patients with signs of secondary T-immunodeficiency the staphylocci antibiotic-sensitivity to one, few or all antibiotics has been increased sharply (more than 100-fold) what permitted further to choose for each patient the antibiotic with exclusively high activity against pathogen. As a whole, within the examined group of patients the reliable decease of MIC of all studied antibiotics has been marked. The proposed treatment regiment permitted to obtain the complete recovery in 27 patients, significant improvement - in 8 patients and moderate improvement - in 1 patient.

### EXAMPLE 33

Thymogen was used in 37 patients with wounds of various origin, type and localization. the control group comprised 24 patients. Thymogen was administered IM or topically single daily at 100 ug for 10 days. The use of Thymogen speeded up (when compared to the control group) significantly wound healing, reduced therapy duration and prevented the development of infectious complications.

### EXAMPLE 34

Administration of Thymogen either intranasally or IM accelerates wound healing, resulting in statistically fewer infections and reduced escar.

### EXAMPLE 35

Thymogen was applied to 44 patients with bone fractures various origin. type and localization. The control group comprised 28 patients. Thymogen was administered intramuscularly or intranasally single daily at 100 up for 10 days. The use of Thymogen accelerated essentially (in comparison with the control group) the consolidation of fractures, prevented the development of infectious complications, reduced pain syndrome and treatment duration.

### EXAMPLE 36

Thymogen was prescribed to 176 patients with chronic osteomyelitis of various ethiology and localization. The control group comprised 88 patients. Thymogen was administered IM or intranasally single daily at 100 ug for 10 days. The use of thymogen rendered the pronounced positive influence on clinical course what expressed in significant decrease of intoxication syndrome and pain syndrome, disappearance of purulent inflammatory manifestations, speeding up of wound healing, reduction of destruction areas, prevention of relapses.

### EXAMPLE 37

A total of 23 patients with cutaneous burns were treated with Thymogen either IM or intranasally with 14 patients for control treated conventionally. Accelerated wound healing, diminished frequency of infections, and less escar was noted in those individuals treated with Thymogen.

### EXAMPLE 38

A total of 17 patients with frostbite to the extremities where treated with Thymogen either IM or intranasally with 11 patient controls. The rapid healing and restoration of tissue integrity was observed.

### EXAMPLE 39

Thymogen administration either IM or intranasally results in less deformity and scarring evidenced by experience in healing fractures, burns, military accidents, and other injuries to the extremities.

### EXAMPLE 40

Experimental data supports the finding that Thymogen administered IM, intranasally, or ocular installation results in restoration and regeneration of corneal epithelium with fewer infections and complications related to escar.

### EXAMPLE 41

A total of 246 patients with various forms of cancer, and 158 controls after radiation and chemotherapy, where Thymogen was administered in single 100 ug daily dose for 10 days experienced normalization of immunological indices, the prevention of post-operative infections, the prevention of upper respiratory infections, and prevention of exacerbations of various secondary complications such as gastritis, cholecystitis, etc. If it was determined necessary based on immunological indices, the treatment regimen was repeated in 4-6 months.

### EXAMPLE 42

Patients treated with Thymogen simultaneously during the administration of chemotherapy experienced fewer complications and side effects related to chemotherapy including diminished frequency and intensity of ulcerative lesions, nausea, and other related problems of chemotherapy administration.

### EXAMPLE 43

Experimental models support the fact that administration of Thymogen prophylactically results in diminished frequency of spontaneous tumorogenesis.

### EXAMPLE 44

Thymogen was applied to 268 persons in combination with the anti-flu vaccination. The control group comprised 197 persons. the vaccination was delivered by air pressure. The Thymogen dose was 50 ug delivered in a single dose for 3 consecutive days. After Thymogen use, it was observed the significant decrease of sickness rate for a period of 12 months compared to controls who received flu-vaccination without Thymogen. In the event of flu, the course of the infection was noted to be less severe and the recovery more rapid when compared to controls.

### EXAMPLE 45

Thymogen was applied in 97 pregnant women with Toxemia of first and second half of pregnancy. The control group comprised 54 patients. Thymogen was administered IM and intranasally at 100 ug daily for 5 - 10 days. Under the influence of Thymogen, it was observed that the BP normalized, and peripheral edema was reduced with normalization of the blood chemistry profile, and the restoration of initially altered immunologic indices.

### EXAMPLE 46

Thymogen was administered to 34 pregnant women with 27 pregnant women for control. The route of administration is IM or intranasally 100 ug daily for 5 - 10 days. Signs of clinical improvement were resolution of weakness, dizziness, and increased appetite, and the normalization of the immunological and hematological indices. It was also noted that there was a decrease in fetal hypoxia.

### EXAMPLE 47

A total of 19 post-term women and 48 women post-term in the control study were treated. The administration of 100 ug of Thymogen IM or intranasally over 3 - 5 days resulted in the effacement of the cervix with thinning at the cervix and the decent of the fetus, with subsequent spontaneous normal delivery.

### EXAMPLE 48

A total of 27 patients with pyelonephritis and 19 control patients with pyelonephritis were treated with the administration of Thymogen single daily dose of 100 ug for 5 - 10 consecutive days in combination with conventional therapy resulted in reduction of fever, the normalization of urina analysis, and the improvement and resolution of the infection. The normal course of delivery in those women treated with Thymogen was without complications.

### EXAMPLE 49

A total of 45 patients with leprosy (Hansen's disease) and 27 infected individuals were treated. Thymogen was administered IM or intranasally in single daily doses of 100 ug for 5 days consecutively in additional to conventional therapy. The patients studied had previous documented resistance to treatment by conventional methods. Thymogen administration resulted in resolution of the lesions and prevented relapses, and promoted more rapid healing of specific ulcers. The immunologic indices were normalized.

### EXAMPLE 50

Thymogen was administered to 84 young sportsmen. The control group consisted of 44 persons. The Thymogen was administered intranasally single dose 1 ug/kg during 3 days. The use of Thymogen resulted in the reduction of upper respiratory infections and rates of illness 4 fold. In the event of infection, it was noted that the infections was less severe without complications, and the clinical improvement was accompanied by the normalization of immunological indices.

### EXAMPLE 51

A total of 33 patients were studied with patients who had relapsing forms of tropical malaria, moderate to severe, and severe cases with 21 patients in the control group. Thymogen was administered at 100 ug single daily doses IM or intranasally for 5 - 10 days. the results of such treatment was reduction of hepatolineal syndrome, the normalization of hematological and immunological indices, reduction of fever, and prevention of relapses.

### EXAMPLE 52

Thymogen was applied in 27 persons with the goal to increase the resistance to excessive solar radiation, in the conditions of hot marine climates. The control group comprised 24 persons. The administration was intranasally 100 ug for 3 days. The use of Thymogen prevented the occurrence of upper respiratory infections in the treated group relative to the control group. There was also noted suppression of their immunologic indices.

### EXAMPLE 53

Thymogen was applied in 21 patients with hemorrhagic Dengue Fever, and 28 patients served as controls. Thymogen was administered IM single daily doses of 100 ug for 5 consecutive days in conjunction with conventional therapy. The results of treatment were reduction in fever, reduction of toxic symptoms, significant decrease in hepato-lineal syndrome. It was also noted that the muscular and bone pain experienced typically was reduced, and the immunological indices were normalized.

### EXAMPLE 54

A total of 48 patients infected and 34 infected controls were examined and treated with administration of Thymogen 100 ug IM or intranasally for 5 - 10 days resulting in normalization of fever, the reduction of toxic symptoms, and the resolution of icterus (jaundice). The hematological and immunological indices were normalized.

### EXAMPLE 55

A total of 36 patients infected and 24 patients infected were controls. Administration of Thymogen in 100 ug IM or intranasally for 5 - 10 days resulted in the reduction of fever, more rapid reduction of toxic symptoms, and the restoration of immunologic indices.

### EXAMPLE 56

A total of 37 patients infected with pulmonary TB and 26 patients infected as controls were studied and treated. Thymogen was administered at 50 to 100 ug every other day during 5 doses total in combination with convention therapy. The results of the treatment 2 months after the course of Thymogen revealed the disappearance of toxic symptoms, the reabsorption of infiltrates, and resolution of pulmonary cavities. The disappearance of TB bacilli was noted in the sputum. The restoration of initially decreased immune indices was also noted.

### EXAMPLE 57

A total of 37 patients, children and adults, with bronchial asthma and 28 similar patients as controls were studied. Thymogen was administered IM single daily doses 1 ug/kg for 5 - 10 days resulting in less severe clinical symptoms. The significant reduction in bronchial obstruction and laryngotracheitis was noted. The normalization of fever, and the reduction in duration of treatment was noted. In some of the patients it was possible to avoid steroids in the conventional commitment treatment course. In the following year observation there was noted a decrease in the incidence of bronchial asthma 4.2 fold. In more than half of the patients the disappearance of drug and food allergy manifestations was noted.

### EXAMPLE 58

A total 125 patients with 53 patients for control infected with Shigella dysentery were examined. Thymogen was administered IM single doses of 100 ug for 10 consecutive days with resultant normalization of fever, the reduction of toxemia, and the normalization gastrointestinal disorders and symptoms. Bacterial shedding in the GI track was observed to cease, and the immunological indices were normalized.

### EXAMPLE 59

A total of 12 patients who had been thymectomized were treated with Thymogen. Prior to therapy these individuals had experienced frequent serious infections including upper respiratory infections. Thymogen was administered in a single dose 100 ug daily for 10 days and repeated every 4 - 6 months. The normalization of immunologic indices was observed, and there was reduction of infectious disorders including cutaneous infections and other chronic exacerbations.

### EXAMPLE 60

A total of 39 patients were studied with 27 patient controls. Thymogen was administered IM or intranasally at 100 up for 5 - 10 days to the study group of patients with the resulting reduction of fever, decrease in toxic symptoms, the reduction of musculoskeletal pain, and the reduction or disappearance of jaundice. Immunological indices were normalized.

### EXAMPLE 61

The use of Thymogen as an ingredient or applicant with cosmetics provides for a less allergenic cosmetic with fewer allergic reactions.

## Claims

1. The use of a peptide for making a pharmaceutical preparation for the treatment of viral infections; wherein the peptide is selected from the group consisting of polymers having the formula:
H₂N--[L-Glu-L-Trp]--ₙ CO₂H;
cyclic polymers of the formula: 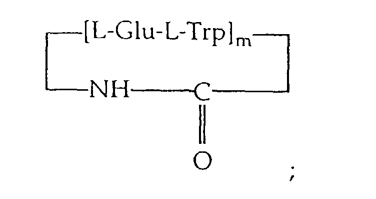 and pharmaceutically acceptable salts thereof, wherein n and m are independently ≥ 2.

2. The use of the peptide according to claim 1 wherein the viral disease is selected from the group consisting of hepatitis, hemorrhagic dengue fever, herpes zoster, influenza, and HIV infection.

3. The use of the peptide according to claim 2 wherein the viral disease is selected from the group consisting of hepatitis A and hepatitis B.

4. The use of the peptide according to claim 1 wherein the viral disease is caused by a virus selected from the group consisting of influenza A and B.

5. The use of a peptide for making a pharmaceutical preparation for the treatment of a mycobacterial infection; wherein the peptide is selected from the group consisting of polymers having the formula:
H₂N--[L-Glu-L-Trp]--ₙ CO₂H;
cyclic polymers of the formula: 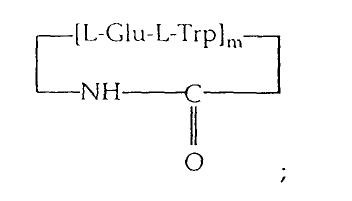 and pharmaceutically acceptable salts thereof, wherein n and m are independently ≥ 2.

6. The use of the peptide according to claim 5 wherein the mycobacterial infection is selected from the group consisting of tuberculosis, and Hansen's disease.

7. The use of a peptide for making a pharmaceutical preparation for the treatment of anemia; wherein the peptide is selected from the group consisting of polymers having the formula:
H₂N --[L-Glu-L-Trp]--ₙ CO₂H;
cyclic polymers of the formula: 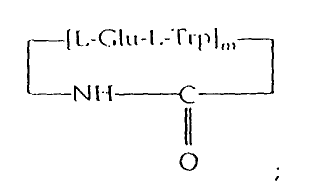 and pharmaceutically acceptable salts thereof, wherein n and m are independently ≥ 2.

8. The use of a peptide for making a pharmaceutical preparation for the treatment of bacterial infections; wherein the peptide is selected from the group consisting of polymers having the formula:
H₂N --[L-Glu-L-Trp]--ₙ CO₂H;
cyclic polymers of the formula: 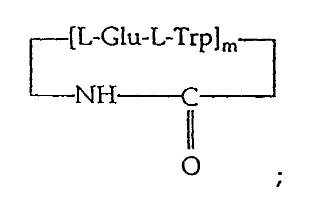 and pharmaceutically acceptable salts thereof, wherein n and m are independently ≥ 2.

9. The use of the peptide according to claim 8 wherein the bacterial disease is caused by a bacteria selected from the group consisting of yersinia, pseudo-tuberculosis, shigella and staphylococci.

10. The use of the peptide according to claim 8 wherein the bacterial disease is selected from the group consisting of apical periodontitis, folliculitis, osteomyclitis, conjunctivitis, uveitis, keratitis, sinusitis, parasinusitis, lymphangitis, periapical granulomas, pelvic inflammatory disease, cervicitis, vaginitis, tubo-ovarian abscess, adnexal abscess, psoriasis, acne vulgaris, eczema pyoderma, furunculitis, cellulitis, pyclonephritis, gingivitis, toxemia and fetal hypoxia.

11. The use according to claims 9 and 10 wherein said pharmaceutical preparation further includes an antibiotic.

12. The use of a peptide for making a pharmaceutical preparation for the treatment of parasitic infection; wherein the peptide is selected from the group consisting of polymers having the formula:
H₂N--[L-Glu-L-Trp]--ₙ CO₂H;
cyclic polymers of the formula: 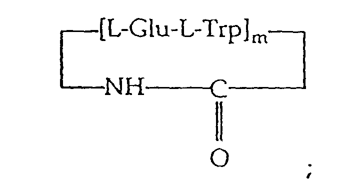 and pharmaceutically acceptable salts thereof, wherein n and m are independently ≥ 2.

13. The use of the peptide according to claim 12 wherein the parasitic disease is malaria.

14. The use of a peptide for making a pharmaceutical preparation for the treatment of an opportunistic infection from AIDS; wherein the peptide is selected from the group consisting of polymers having the formula:
H₂N--[L-Glu-L-Trp]--ₙ CO₂H;
cyclic polymers of the formula: 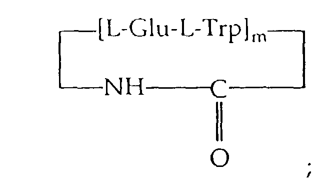 and pharmaceutically acceptable salts thereof, wherein n and m are independently ≥ 2.

15. The use of the peptide according to claim 14 wherein the opportunistic infection is selected from the group consisting of fungal infections, bacterial infections and viral infections.

16. The use of a peptide for making a pharmaceutical preparation for the treatment of hyper immune conditions; wherein the peptide is selected from the group consisting of polymers having the formula:
H₂N--[L-Glu-L-Trp]--ₙCO₂H;
cyclic polymers of the formula: 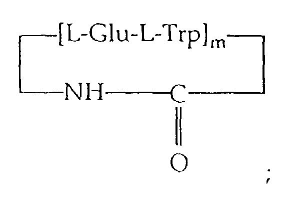 and pharmaceuticlaly acceptable salts thereof, wherein n and m are independently ≥ 2.

17. The use of the peptide according to claim 18 wherein the hyper immune condition is selected from the group consisting of bronchial asthma, drug allergy, food allergy, allergy to skin contact with chemicals used in cosmetics, allergy to antibiotics, and rheumatoid arthritis.

18. The use of a peptide for making a pharmaceutical preparation for delivering in combination with vaccination for the prophylaxis of viral infections; wherein the peptide is selected from the group consisting of polymers having the formula:
H₂N--[L-Glu-L-Trp]--ₙCO₂H;
cyclic polymers of the formula: 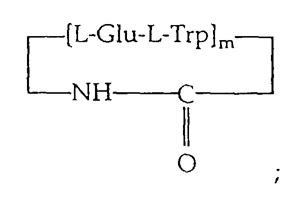 and pharmaceutically acceptable salts thereof, wherein n and m are independently ≥ 2.

19. The use of a peptide for making a pharmaceutical preparation for the treatment of soft tissue damage caused by environmental exposure; wherein the peptide is selected from the group consisting of polymers having the formula:
H₂N--[L-Glu-L-Trp]--ₙ CO₂H;
cyclic polymers of the formula: 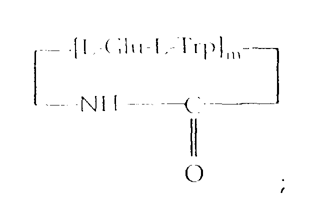 and pharmaceutically acceptable salts thereof, wherein n and m are independently ≥ 2.

20. The use of the peptide according to claim 19 wherein the soft tissue damage is selected from the group consisting of frostbite, thermal burns, and radiation burns.

21. The use of a peptide for making a pharmaceutical preparation for skin grafting; wherein the peptide is selected from the group consisting of polymers having the formula:
H₂N--[L-Glu-L-Trp]--ₙCO₂H;
cyclic polymers of the formula: 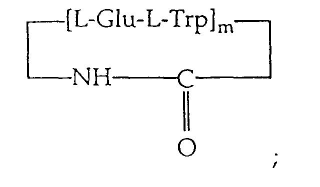 and pharmaceutically acceptable salts thereof, wherein n and m are independently ≥ 2.

22. A pharmaceutical preparation comprising a therapeutically effective amount to lower a hyperactive immune state of polymers of the formula: 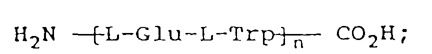 cyclic polymers of the formula: 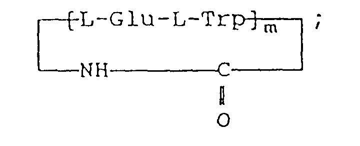 and/or their pharmaceutically acceptable salts thereof in a pharmaceutically acceptable vehicle, wherein n and m are independently ≥2.

23. A pharmaceutical preparation for the restoration of normal immunological indices comprising a therapeutically effective amount to restore normal immunological indices in a subject of polymers of the formula: 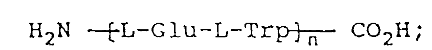 cyclic polymers of the formula: 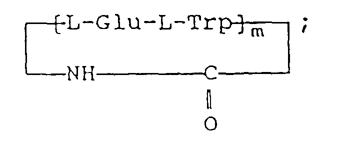 and/or their pharmaceutically acceptable salts thereof in a pharmaceutically acceptable vehicle, wherein n and m are independently ≥2.

24. A pharmaceutical preparation according to Claims 22 or 23 in the form of an injectable solution containing .001 to .01% by weight of said polymer.

25. A pharmaceutical preparation according to Claim 22 or 23 in the form of tablets, suppositories, capsules, eye films, inhalant, mucosal spray, toothpaste, ointments, and/or water soluble based creams.

26. A pharmaceutical preparation according to Claim 22 wherein said tablets, suppositories, capsules, eye films, inhalants, mucosal sprays, toothpaste, ointments, and/or water soluble based creams contain at least 0.01 mg of said dipeptide per unit preparation.

27. A substantially pure compound selected from the group consisting of polymers of the formula: 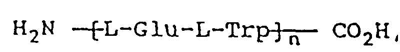 cyclic polymers of the formula 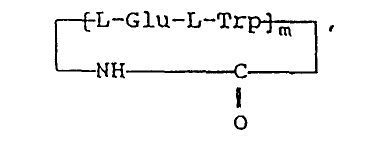 and their pharmaceutically acceptable salts; wherein n and m are independently ≥2.

28. The use of a peptide selected from polymers of the formula:
H₂N--[L-Glu--Trp]--ₙCO₂H;
all cyclic polymers of the formula: 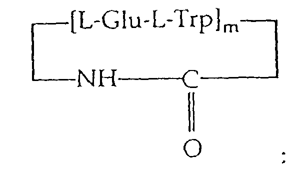 and pharmaceutically acceptable salts thereof wherein n and m are independently greater than or equal to 2 for making a pharmaceutical preparation for the treatment of hypotrophy in non-human animals including fur-bearing animals and poultry.

## Patentansprüche

1. Verwendung eines Peptids zur Herstellung einer pharmazeutischen Zusammensetzung zur Behandlung viraler Infektionen, wobei das Peptid ausgewählt ist aus der Gruppe, die gebildet wird von Polymeren der Formel:
H₂N--[L-Glu-L-Trp]ₙ-CO₂H;
zyklischen Polymeren der Formel: 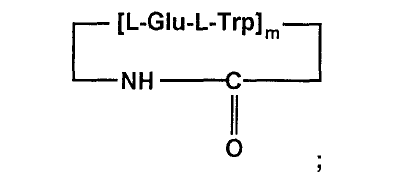 sowie deren pharmazeutisch verträglichen Salzen, wobei n und m unabhängig voneinander ≥ 2 sind.

2. Verwendung des Peptids nach Anspruch 1, wobei die virale Infektion ausgewählt ist aus der Gruppe, die gebildet wird von Hepatitis, hämmorhagischem Denguefiber, Herpes Zoster, Grippe und HIV-Infektionen.

3. Verwendung des Peptids nach Anspruch 2, wobei die virale Infektion ausgewählt ist aus der Gruppe, die von Hepatitis A und Hepatitis B gebildet wird.

4. Verwendung des Peptids nach Anspruch 1, wobei die virale Infektion durch einen Virus verursacht ist, der ausgewählt ist aus der Gruppe, die gebildet ist von Influenza A und B.

5. Verwendung eines Peptids zur Herstellung einer pharmazeutischen Zusammensetzung zur Behandlung einer mykobakteriellen Infektion, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 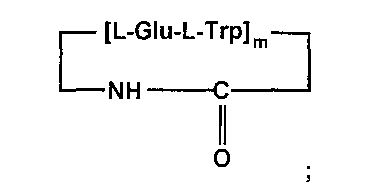 sowie deren pharmazeutisch verträglichen Salzen, wobei n und m unabhängig voneinander ≥ 2 sind.

6. Verwendung des Peptids nach Anspruch 5, wobei die mykobakterielle Infektion ausgewählt ist aus der Gruppe, die gebildet ist von Tuberkulose und Lepra (Hansens Krankheit).

7. Verwendung eines Peptids zur Herstellung einer pharmazeutischen Zusammensetzung zur Behandlung von Anämie, wobei das Peptid ausgewählt ist aus der Gruppe, die gebildet ist von Polymeren der Formel:
H₂N--[L-Glu-L-Trp]ₙ-CO₂H;
zyklischen Polymeren der Formel : 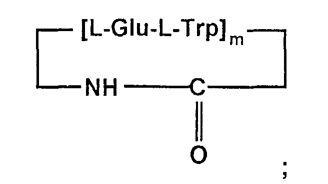 sowie deren pharmazeutisch verträglichen Salzen, wobei n und m unabhängig voneinander ≥ 2 sind.

8. Verwendung eines Peptids zur Herstellung einer pharmazeutischen Zusammensetzung zur Behandlung bakterieller Infektionen, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 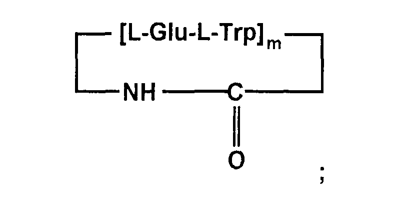 sowie deren pharmazeutisch verträglichen Salzen, wobei n und m unabhängig voneinander ≥ 2 sind.

9. Verwendung eines Peptids nach Anspruch 8, wobei die bakterielle Infektion verursacht wird von Bakterien, die ausgewählt sind aus der Gruppe, die gebildet ist von Yersinien, pseudo-Tuberkulosis, Shigellen und Staphylokokken.

10. Verwendung des Peptids nach Anspruch 8, wobei die bakterielle Erkrankung ausgewählt ist aus der Gruppe, die gebildet ist von apikaler Periodontitis, Follikulitis, Osteomyelitis, Conjunktivitis, Uveitis, Keratitis, Sinusitis, Parasinusitis, Lymphangitis, periapikalen Granulomen, Entzündungen im Beckenraum, Cervicitis, Vaginitis, tuboovariellem Abszess, Abszessen der Adnexe, Psoriasis, Akne vulgaris, Ekzemen, Pyodermie, Furunkulitis, Cellulitis, Pyelonephritis, Gingivitis, Toxemie und fetaler Hypoxie.

11. Verwendung nach Anspruch 9 oder 10, wobei die pharmazeutische Zusammensetzung weiter ein Antibiotikum enthält.

12. Verwendung eines Peptids zur Herstellung einer pharmazeutischen Zusammensetzung zur Behandlung parasitischer Infektionen, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 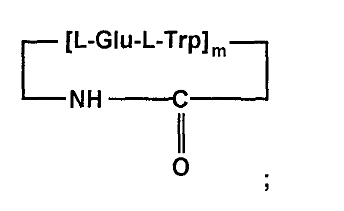 sowie deren pharmazeutisch verträglichen Salzen, wobei n und m unabhängig voneinander ≥ 2 sind.

13. Verwendung nach Anspruch 12, wobei die parasitische Infektion Malaria ist.

14. Verwendung eines Peptids zur Herstellung einer pharmazeutischen Zubereitung zur Behandlung opportunistischer Infektionen bei AIDS, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 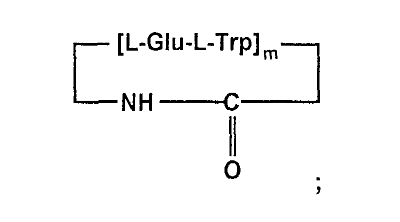 sowie deren pharmazeutisch verträglichen Salzen, wobei n und m unabhängig voneinander ≥ 2 sind.

15. Verwendung des Peptids nach Anspruch 14, wobei die opportunistische Infektion ausgewählt ist aus der Gruppe, die gebildet ist von Pilzinfektionen, bakteriellen Infektionen und viralen Infektionen.

16. Verwendung eines Peptids zur Herstellung einer pharmazeutischen Zusammensetzung zur Behandlung von hyperimmunen Zuständen, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 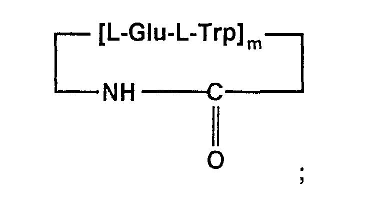 sowie deren pharmazeutisch verträglichen Salzen, wobei n und m unabhängig voneinander ≥ 2 sind.

17. Verwendung des Peptids nach Anspruch 18, wobei der hyperimmune Zustand ausgewählt ist aus der Gruppe, die gebildet wird von bronchialem Asthma, Arzneimittelallergie, Nahrungsmittelallergie, Hautkontaktallergien gegen Chemikalien, die in Kosmetika verwendet werden, Allergien gegen Antibiotika und rheumatoide Arthritis.

18. Verwendung eines Peptids zur Herstellung einer pharmazeutischen Zusammensetzung für die kombinierte Gabe mit einer Impfung zur Prophylaxe gegen virale Infektionen, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 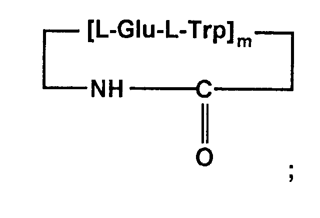 sowie deren pharmazeutisch verträglichen Salzen, wobei n und m unabhängig voneinander ≥ 2 sind.

19. Verwendung eines Peptids zur Herstellung einer pharmazeutischen Zusammensetzung für die Behandlung von Gewebeschädigungen durch Umwelteinflüsse, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 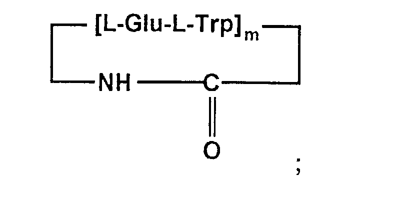 sowie deren pharmazeutisch verträglichen Salzen, wobei n und m unabhängig voneinander ≥ 2 sind.

20. Verwendung des Peptids nach Anspruch 19, wobei die Gewebeschädigung ausgewählt ist aus der Gruppe, die gebildet ist von Erfrierungen, thermischen Verbrennungen und Verbrennungen durch radioaktive Strahlung.

21. Verwendung eines Peptids zur Herstellung einer pharmazeutischen Zusammensetzung für die Verpflanzung von Haut, wobei das Peptid ausgewählt ist aus der Gruppe, die gebildet ist von Polymeren der Formel:
H₂N--[L-Glu-L-Trp]ₙ-CO₂H;
zyklischen Polymeren der Formel: 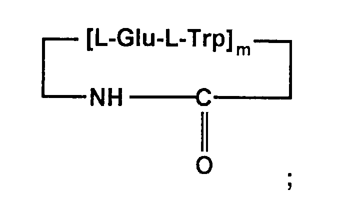 sowie deren pharmazeutisch verträglichen Salzen, wobei n und m unabhängig voneinander ≥ 2 sind.

22. Pharmazeutische Zusammensetzung, enthaltend eine zur Erniedrigung eines hyperaktiven Immunzustands therapeutisch wirksame Menge eines Polymeren der Formel:
H₂N--[L-Glu-L-Trp]ₙ-CO₂H;
eines zyklischen Polymeren der Formel: 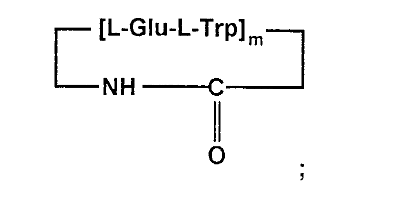 und/oder eines ihrer pharmazeutisch verträglichen Salze, in einem pharmazeutisch verträglichen Träger, wobei n und m unabhängig voneinander ≥ 2 sind.

23. Pharmazeutische Zubereitung zur Wiederherstellung normaler immunologischer Werte, enthaltend eine zur Wiederherstellung normaler immunologischer Werte in einem Subjekt therapeutisch wirksame Menge eines Polymeren der Formel:
H₂N--[L-Glu-L-Trp]ₙ-CO₂H;
eines zyklischen Polymeren der Formel: 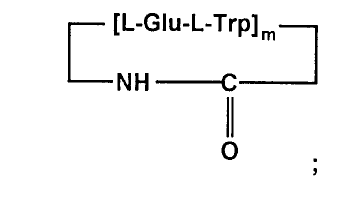 und/oder eines ihrer pharmazeutisch verträglichen Salze, in einem pharmazeutisch verträglichen Träger, wobei n und m unabhängig voneinander ≥ 2 sind.

24. Pharmazeutische Zubereitung nach Anspruch 22 oder 23 in Form einer injizierbaren Lösung, die 0,001 bis 0,1 Gew.-% des Polymeren enthält.

25. Pharmazeutische Zubereitung nach Anspruch 22 oder 23 in Form von Tabletten, Suppositorien, Kapseln, Augentropfen, Inhalationsmitteln, Schleimhautsprays, Zahnpasta, Salben und/oder auf Wasser basierenden Cremes.

26. Pharmazeutische Zubereitung nach Anspruch 22, wobei die Tabletten, Suppositorien, Kapseln, Augentropfen, Inhalationsmittel, Schleimhautsprays, Zahnpasta, Salben und/oder auf Wasser basierenden Cremes wenigstens 0,01 mg des Dipeptids pro Verabreichungseinheit enthalten.

27. Im wesentlichen reine Verbindung, ausgewählt aus der Gruppe, die gebildet ist von Polymeren der Formel
H₂N--[L-Glu-L-Trp]ₙ-CO₂H;
zyklischen Polymeren der Formel: 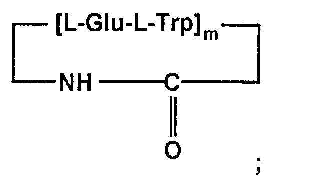 und/oder eines ihrer pharmazeutisch verträglichen Salze, in einem pharmazeutisch verträglichen Träger, wobei n und m unabhängig voneinander ≥ 2 sind.

28. Verwendung eines Peptids, ausgewählt aus Polymeren der Formel:
H₂N--[L-Glu-L-Trp]ₙ-CO₂H;
aller zyklischen Polymeren der Formel: 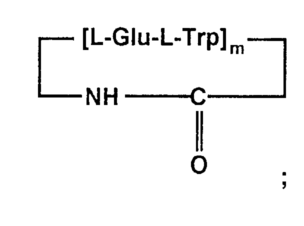 und ihre pharmazeutisch verträglichen Salze, wobei n und m unabhängig voneinander größer oder gleich 2 ist, zur Herstellung pharmazeutischer Zubereitungen zur Behandlung von Hypotrophie in nicht-menschlichen Tieren, einschließlich pelztragenden Tieren und Geflügel.

## Revendications

1. L'utilisation d'un peptide pour la fabrication d'une préparation pharmaceutique pour le traitement d'infections virales, dans laquelle le peptide est choisi dans le groupe consistant en les polymères répondant à la formule :
H₂N-[L-Glu-L-Trp]ₙ-CO₂H;
les polymères cycliques de formule : 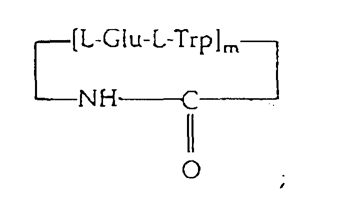 et leurs sels pharmaceutiquement acceptables, où n et m sont indépendamment ≥ 2.

2. L'utilisation d'un peptide selon la revendication 1, dans laquelle la maladie virale est choisie dans le groupe consistant en l'hépatite; la dengue à forme hémorragique, le zona herpétique, la grippe, et l'infection par le VIH.

3. L'utilisation d'un peptide selon la revendication 2, dans laquelle la maladie virale est choisie dans le groupe consistant en hépatite A et hépatite B

4. L'utilisation d'un peptide selon la revendication 1, dans laquelle la maladie virale est provoquée par un virus choisi dans le groupe consistant en grippe A et B.

5. L'utilisation d'un peptide pour la fabrication d'une préparation pharmaceutique pour le traitement d'une infection mycobactérienne, dans laquelle le peptide est choisi parmi les polymères répondant à la formule :
H₂N-[L-Glu-L-Trp]ₙ-CO₂H;
les polymères cycliques de formule : 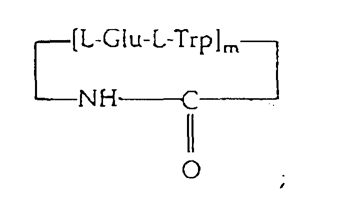 et leurs sels pharmaceutiquement acceptables, où n et m sont indépendamment ≥ 2.

6. L'utilisation d'un peptide selon la revendication 5, dans laquelle l'infection mycobactérienne est choisie dans le groupe consistant en tuberculose et maladie de Hansen.

7. L'utilisation d'un peptide pour la fabrication d'une préparation pharmaceutique pour le traitement de l'anémie, dans laquelle le peptide est choisi dans le groupe consistant en les polymères répondant à la formule :
H₂N-[L-Glu-L-Trp]ₙ-CO₂H;
les polymères cycliques de formule : 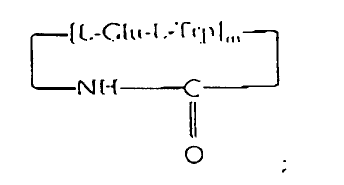 et leurs sels pharmaceutiquement acceptables, où n et m sont indépendamment ≥ 2.

8. L'utilisation d'un peptide pour la fabrication d'une préparation pharmaceutique pour le traitement d'infections bactériennes, dans laquelle le peptide est choisi dans le groupe consistant en les polymères répondant à la formule :
H₂N-[L-Glu-L-Trp]ₙ-CO₂H;
les polymères cycliques de formule : 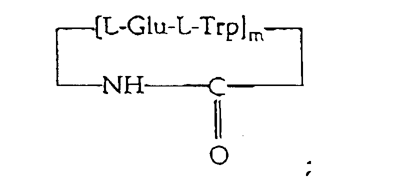 et leurs sels pharmaceutiquement acceptables, où n et m sont indépendamment ≥ 2.

9. L'utilisation du peptide selon la revendication 8, dans laquelle la maladie bactérienne est provoquée par une bactérie choisie dans le groupe consistant en *yersinia, pseudo-tuberculosis, shigella* et *staphylococci.*

10. L'utilisation du peptide selon la revendication 8, dans laquelle la maladie bactérienne est choisie dans le groupe consistant en *periodontitis* apicale, folliculitis, *osteomyelitis, conjunctivitis, uveitis, keraritis, sinusitis, parasinusitis, lumphangitis, granulomas* periapicale, maladie inflammatoire pelvienne, *cervicitis, vaginitis,* abcès tubo-ovarien, abcès adnexiel, *psoriasis, acné vulgaris, eczéma, pyoderma, furonculitis, cellulitis, pyelonephritis, gingivitis,* toxémie et hypoxie foetale.

11. L'utilisation selon l'une quelconque des revendications 9 ou 10, dans laquelle ladite préparation pharmaceutique comprend en outre un antibiotique.

12. L'utilisation d'un peptide pour la fabrication d'une préparation pharmaceutique pour le traitement d'infections parasitaires, dans laquelle le peptide est choisi dans le groupe consistant en les polymères répondant à la formule :
H₂N-[L-Glu-L-Trp]ₙ-CO₂H;
les polymères cycliques de formule : 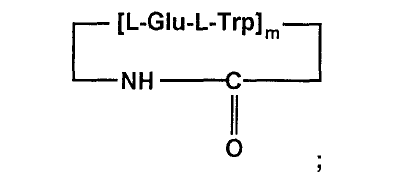 et leurs sels pharmaceutiquement acceptables, où n et m sont indépendamment ≥ 2.

13. L'utilisation d'un peptide selon la revendication 12, dans laquelle la maladie parasitaire est la malaria.

14. L'utilisation d'un peptide pour la fabrication d'une préparation pharmaceutique pour le traitement d'une infection opportuniste du SIDA, dans laquelle le peptide est choisi dans le groupe consistant en les polymères répondant à la formule :
H₂N-[L-Glu-L-Trp]ₙ-CO₂H;
les polymères cycliques de formule : 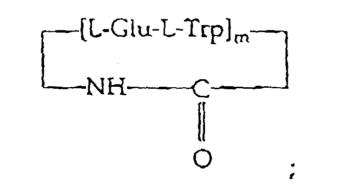 et leurs sels pharmaceutiquement acceptables, où n et m sont indépendamment ≥ 2.

15. L'utilisation d'un peptide selon la revendication 14, dans laquelle l'infection opportuniste est choisie dans le groupe consistant en infections fongiques, infections bactériennes et infections virales.

16. L'utilisation d'un peptide pour la fabrication d'une préparation pharmaceutique pour le traitement d'états hyper-immuns, dans laquelle le peptide est choisi dans le groupe consistant en les polymère répondant à la formule :
H₂N-[L-Glu-L-Trp]ₙ-CO₂H;
lcs polymères cycliques de formule : 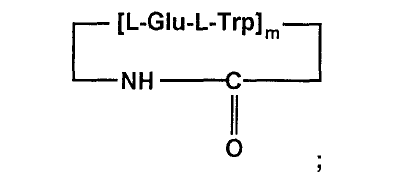 et leurs sels pharmaceutiquement acceptables, où n et m sont indépendamment ≥ 2.

17. L'utilisation d'un peptide selon la revendication 16, dans laquelle l'état hyper-immun est choisi dans le groupe consistant en asthme bronchique, allergie médicamenteuse, allergie alimentaire, allergie au contact cutané avcc les produits chimiques utilisés dans les cosmétiques, allergie aux antibiotiques, et arthrite rhumatoïde.

18. L'utilisation d'un peptide pour la fabrication d'une préparation pharmaceutique pour la libération en combinaison avec la vaccination pour la prophylaxie d'infections virales, dans laquelle le peptide est choisi dans le groupe consistant en les polymères répondant à la formule :
H₂N-[L-Glu-L-Trp]ₙ-CO₂H;
les polymères cycliques de formule : 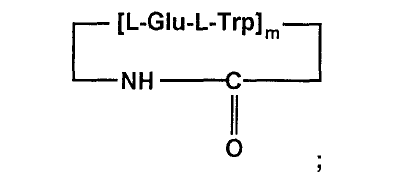 et leurs sels pharmaceutiquement acceptables, où n et m sont indépendamment ≥ 2.

19. L'utilisation d'un peptide pour la fabrication d'une préparation pharmaceutique pour le traitement de lésions des tissus mous provoquées par une exposition à l'environnement, dans laquelle le peptide est choisi dans le groupe consistant en les polymère répondant à la formule :
H₂N-[L-Glu-L-Trp]ₙ-CO₂H;
les polymères cycliques de formule : 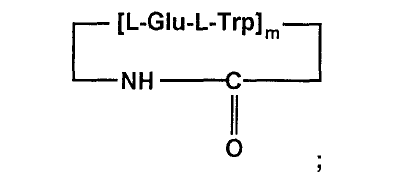 et leurs sels pharmaceutiquement acceptables, où n et m sont indépendamment ≥ 2.

20. L'utilisation du peptide selon la revendication 19, dans laquelle la lésion du tissu mou est choisie dans le groupe consistant en engelures, brûlures thermiques et brûlures par irradiation.

21. L'utilisation d'un peptide pour la fabrications d'une préparation pharmaceutique pour la greffe cutanée, dans laquelle le peptide est choisi dans le groupe consistant en les polymères répondant à la formule :
H₂N-[L-Glu-L-Trp]ₙ-CO₂H;
les polymères cycliques de formule : 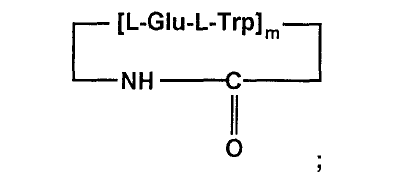 et leurs sels pharmaceutiquement acceptables, où n et m sont indépendamment ≥ 2.

22. Une préparation pharmaceutique comprenant une quantité thérapeutiquement efficace pour abaisser un état immun hyperactif, de polymères de formule :
H₂N-[L-Glu-L-Trp]ₙ-CO₂H;
de polymères cycliques de formule : 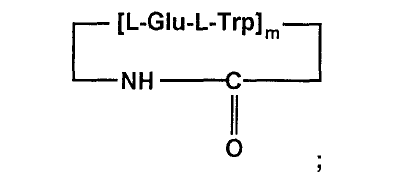 et/ou ou leurs sels pharmaceutiquement acceptables dans un véhicule pharmaceutiquement acceptable, où n et m sont indépendamment ≥ 2.

23. Une préparation pharmaceutique pour la restauration des indices immunologiques normaux comprenant une quantité thérapeutiquement efficace pour restaurer les indices immunologiques normaux chez un patient, de polymères de formule :
H₂N-[L-Glu-L-Trp]ₙ-CO₂H;
de polymères cycliques de formule : 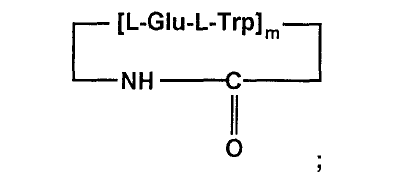 et/ou ou leurs sels pharmaceutiquement acceptables dans un véhicule pharmaceutiquement acceptables où n et m sont indépendamment ≥ 2.

24. Une préparation pharmaceutique selon une quelconque des revendications 22 ou 23, sous forme d'une solution injectable contenant de 0,001 à 0,01% en poids dudit polymère.

25. Une préparation pharmaceutique selon l'une quelconque des revendications 22, 23, sous forme de comprimés, suppositoires, capsules, films oculaires, inhalations, pulvérisations mucosales, pâtes dentifrices pommades et/ou crèmes à base hydrosoluble.

26. Une préparation pharmaceutique selon la revendication 22, dans laquelle lesdits comprimés, capsules, films oculaires, inhalations, pulvérisations mucosales, pâtes dentifrices, pommades et/ou crèmes à base hydrosoluble contiennent au moins 0.01 mg dudit peptide par préparation unitaire.

27. Un composé sensiblement pur choisi dans le groupe consistant en polymères de formule :
H₂N-[L-Glu-L-Trp]ₙ-CO₂H;
polymères cycliques de formule : 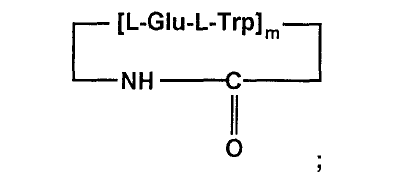 et leurs sels pharmaceutiquement acceptables, où n et m sont indépendamment ≥ 2.

28. L'utilisation d'un peptide choisi parmi les polymères de formule:
H₂N-[L-Glu-L-Trp]ₙ-CO₂H;
tous les polymères cycliques de formule : 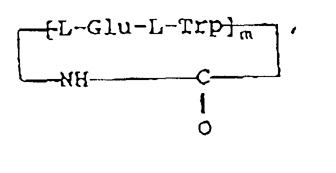 et leurs sels pharmaceutiquement acceptables, où n et m sont indépendamment ≥ 2, pour la fabrication d'une préparation pharmaceutique pour le traitement de l'hypotrophie chez les animaux non humains, y compris les animaux à fourrure et la volaille.
